# EUROPÄISCHE PATENTSCHRIFT

(11) **EP 1 450 763 B1**
(45) Veröffentlichungstag und Bekanntmachung des Hinweises auf die Patenterteilung: **16.02.2011**
(21) Anmeldenummer: 02804214.1
(22) Anmeldetag: 03.12.2002
(51) Int. Cl.: A61K 9/00, A61K 47/30

(54) **MITTEL ZUR BEHANDLUNG VON ÜBERGEWICHT IN KOMBINATION MIT WEITEREN INDIKATIONEN**
AGENT FOR TREATING EXCESS WEIGHT IN ASSOCIATION WITH OTHER INDICATIONS
AGENT POUR TRAITER LA SURCHARGE PONDERALE EN ASSOCIATION AVEC D'AUTRES INDICATIONS

(30) Priorität: 06.12.2001 DE 10164680
(43) Veröffentlichungstag der Anmeldung: 01.09.2004
(73) Patentinhaber: Schütte/Reichel GbR, 40668 Meersbusch (DE)
(72) Erfinder: Beisel Günther, 50667 Köln (DE)
(74) Vertreter: Fitzner, Uwe
(86) Internationale Anmeldenummer: PCT/EP2002/013634
(87) Internationale Veröffentlichungsnummer: WO 2003/047546

(56) Entgegenhaltungen:
- EP-A- 1 029 892
- WO-A-01/17377
- DE-U- 20 119 843
- US-A- 4 869 908
- US-A- 5 010 061

## Beschreibung

Gegenstand der vorliegenden Erfindung ist ein Mittel zur Behandlung von Übergewicht in Kombination mit mindestens einer weiteren Indikation sowie ein Verfahren zu dessen Herstellung und dessen Verwendung.

Arzneimittel mit zeitlich gesteuerter Wirkstofffreisetzung im Gastrointestinaltrakt sind seit mehreren Jahrzehnten bekannt. Der Wirkstoff wird aus unterschiedlich aufgebauten Darreichungsformen durch Löse-, Erosions- oder Diffusionsprozesse während der Passage des Gastrointestinaltrakts freigesetzt.

Arzneimittel mit verlängerter Magenverweilzeit, die aus expandierenden Trägermaterialien bestehen, werden in DE 33 447 72 beschrieben. Nach oraler Einnahme entfalten sich papierartige Arzneiträger im Magen. Aufgrund ihrer Größe erfolgt ein verzögerter Weitertransport vom Magen in den Darm. Der Wirkstoff wird zeitlich gesteuert im Magen freigesetzt. Die Resorption des gelösten Wirkstoffs erfolgt aus dem oberen Dünndarm.

In US 4,126,672, US 4,140,755 und US 4,814,179 werden Schwimmarzneiformen beschrieben, die aufgrund ihrer geringen Dichte auf dem Mageninhalt aufschwimmen und die erst nach vollständiger Entleerung des Magens weitertransportiert werden. Hydrokolloide und hydrophobe Substanzen bewirken das Aufschwimmen des Kapselinhalts. Beispielhaft seien hier Levodopa- Retardkapseln genannt.

Die Freisetzung von Wirkstoffen aus schwammartigen Arzneiträgern wird in WO 98/09617 beschrieben. Durch die Expansion der schwammartigen Materialien in Flüssigkeiten im Magen-Darm-Trakt ist eine Beeinflussung der Magenverweilzeit zu erwarten. Gleichzeitig wird über die Oberflächenvergrößerung eine Freisetzung der Wirkstoffe unter größtmöglicher Schonung der Schleimhäute erhalten.

In DE 197 39 031 wird die Kombination von komprimiertem Kollagen mit einer Retardschicht aus Hydroxypropylmethycellulose und Arzneistoff angegeben. Die Herstellung erfolgt wie bei Zweischichtentabletten durch einen zweistufigen Pressvorgang.

Diese bekannten Mittel beziehen sich darauf, dass die Verweilzeit im Magen erhöht wird. Das Problem, dass im einzelnen Fall Wirkstoffe schnell in den Darm gelangen sollen, während andererseits ein Teil des Mittels im Magen verbleiben soll wird in dem beschriebenen Stand der Technik nicht erörtert.

WO 01/17377 A1 und EP 1 029 892 A2 offenbaren volumenvergrößernde Materialien und wirkstoffhaltigen Formulierungen, die jedoch nicht über speziell ausgestaltete Haftvermittler verbunden sind.

Aufgabe der vorliegenden Erfindung ist es deshalb, ein Mittel zur Verfügung zu stellen, das zur Behandlung von Übergewicht und zugleich mindestens einer weiteren Indikation geeignet ist.

Diese Aufgabe wird erfindungsgemäß durch ein Mittel zur Behandlung von Übergewicht in Kombination mit mindestens einer weiteren Indikation enthaltend ein in gastrointestinalen Flüssigkeiten und/oder Körperflüssigkeiten unlösliches oder schwer lösliches, volumenvergrößerndes Material und wenigstens eine wirkstoffhaltige im Darm resorbierbare Formulierung, die mittels eines im Magen zumindest teilweise löslichen Haftvermittlers derart miteinander verbunden sind, daß
a) unter den Milieubedingungen im Magen das Material und die Formulierung voneinander getrennt werden,
b) aufgrund dessen die Formulierung in den Darm gelangt, während das volumenvergrößernde Material im Magen verbleibt, wobei
   als Haftvermittler Stärkederivate, Cellulosederivate, Gelatine oder Polysaccharide eingesetzt werden,
   als volumenvergößerndes Material feste oder halbfeste elastische Schäume, die aus gasgefüllten Zellen bestehen, die durch hochviskose und/oder feste Zellstege begrenzt sind, ausgewählt aus der Gruppe bestehend aus natürlich vorkommenden Schwämme, halbsynthetischen oder synthetisch hergestellten schwammartigen Gebilden, eingesetzt werden, und
   als im Darm resorbierbare Formulierung wirkstoffhaltige Formulierungen, enthaltend Wirkstoffe in Form eines Pulvers, einer Paste, einer Folie, als Lösung, als Schmelze, eingesetzt werden, und wobei
   durch Beaufschlagen der wirkstoffhaltigen Formulierung und/oder des Trägermaterials mit dem Haftvermittler eine Verbindung zwischen volumenvergrößerndem Material und der wirkstoffhaltigen Schicht erzielt wird, die Haftvermittlerschicht folienförmig ausgestaltet ist und/oder der Haftvermittler fadenförmige und/oder gewebeähnliche Stoffe enthält.

Es ist aber auch möglich, zusätzlich zu der Haftvermittlungsreaktion eine Umhüllung vorzusehen.

Durch das Beaufschlagen der wirkstoffhaltigen Formulierung und/oder des Trägermaterials mit dieser verbindenden Schicht wird eine Verbindung zwischen gegebenenfalls komprimierten und im Gastrointestinaltrakt volumenvergrößerenden Trägermaterial und der wirkstoffhaltigen Schicht erzielt. Die Dauer der bestehenden Verbindung hängt dabei von der Löslichkeit der eingesetzten und in der verbindenden Schicht enthaltenen Stoffe im Magensaft und/oder anderen gastrointestinalen Flüssigkeiten ab.

In der vorliegenden Erfindung handelt es sich bei dem Haftvermittler um Stärkederivate, Cellulosederivate, Gelatine oder Polysaccharide, vorzugsweise polyuronsäurehaltige Polysaccharide, z.B. Polyalginate. Beispielhaft seien Hypomellose, Carmellose, Metholose genannt.

Besonders bevorzugte Polyuronsäure-haltige Polysaccharide sind Alginsäuren und deren Salze (Alginate). Aber auch niederveresterte Pectine, Xanthan, Tragant, Chondroitinsulfat sowie alle anderen Uronsäure-haltigen Verbindungen können erfindungsgemäß zum Einsatz kommen.

Alginsäure ist eine lineare Polyuronsäure aus wechselnden Anteilen von D-Mannuronsäure und L-Guluronsäure, die durch beta-glykosidische Bindungen miteinander verknüpft sind, wobei die Carboxylgruppen nicht verestert sind. Ein Molekül Alginsäure kann sich aus etwa 150-1050 Uronsäure-Einheiten zusammensetzen, wobei das durchschnittliche Molekulargewicht in einem Bereich von 30-200 kDa variieren kann.

Das Polysaccharid Alginsäure ist ein Bestandteil der Zellwänden von Braunalgen. Der Anteil der Alginsäure an der Trockenmasse der Algen kann hierbei bis zu 40% ausmachen. Die Gewinnung der Alginsäure erfolgt durch alkalische Extraktion mit an sich bekannten Methoden gemäß dem Stand der Technik. Die resultierende pulverförmige Alginsäure ist somit rein pflanzlich und weist eine hohe Biokompatibilität auf. Sie kann unter Bildung hochviskoser Lösungen die 300-fache Menge ihres Eigengewichtes an Wasser aufnehmen. In Gegenwart von mehrwertigen Kationen bildet Alginsäure sogenannte Gele. Die Bildung von Alginatgelen in Gegenwart zweiwertiger Kationen, wie Calcium oder Barium, sind bei Shapiro I., et al. (Biomaterials, 1997, 18: 583-90) beschrieben. Letzteres ist aufgrund seiner Toxizität für den Einsatz in Biomedizin jedoch nicht geeignet. Neben Calcium-Chlorid liefert auch Calcium-Glukonat geeignete zweiwertige Kationen. Generell sind alle physiologisch unbedenklichen Poly-Kationen, insbesondere zweiwertige Kationen verwendbar.

Das erfindungsgemäße Mittel kann beispielsweise in Form von Tabletten, Kapseln, Dragees, als Granulat oder Zäpfchen oder anderen Ausgestaltungen vorliegen. Darüber hinaus kann das erfindungsgemäße Mittel als eine äußere Schicht einen Überzug aufweisen. Dies kann eine Lackschicht, Filmüberzug oder andere Schicht sein, die die Einnahme des erfindungsgemäßen Mittels erleichtert, den Inhaltsstoff zusammenhält und die sich erst im Gastrointestinaltrakt, beispielsweise unter Einfluß der Magenflüssigkeit, auflöst.

Die Beschichtung der volumenvergrößernden Materialien und wirkstoffhaltigen Formulierungen kann durch Tränken der Formlinge ggf. bei höheren Temperaturen erfolgen. Die hydrophilen oder lipophilen Hilfsstoffe können flüssig oder fest sein. Beispiele für Filmüberzüge sind Cellulosederivate oder Polyacrylate ggf. mit speichelresistenten Eigenschaften. Hydrophile Hilfsstoffe sind Glycerol, Propylenglycol oder Macrogole. Hydrophobe Hilfsstoffe sind Wachse, Fette oder Öle.

In einer bevorzugten Ausführungsform der Erfindung werden die volumenvergrößernden Materialien und die wirkstoffhaltigen Formulierungen in Behältnisse eingebracht. Als Behältnisse werden Kapseln in unterschiedlicher Form und aus unterschiedlichen Materialien verwendet. Ebenso können Behältnisse durch Aufpressen von flachen folienartigen Materialien aus Polymeren oder Faserstoffen erzeugt werden.

Das erfindungsgemäße Mittel wird nach der Einnahme durch den Patienten im Magen in volumenvergrößerndes Trägermaterial und die wirkstoffhaltige Formulierung(en) enthaltende(n) Schicht(en) aufgespalten bzw. getrennt, sobald die Umhüllung und/oder der Haftvermittler sich wenigstens teilweise unter den Milieubedingungen im Magen gelöst haben. Die wirkstoffhaltige(n) Formulierung(en) kann (können) aufgrund dessen in den Darm gelangen, d.h. aufgrund dessen im Darm ungehindert resorbiert werden, während das volumenvergrößernde Material sättigend im Magen verbleibt.

Das volumenvergrößernde Material gewährleistet die Sättigungswirkung, da es aufgrund der erforderlichen Volumenvergrößerung größer als die Öffnung des Magenausgangs ist und somit an einem raschen Weitertransport vom Magen in den Darm gehindert wird, d.h. die Verweilzeit im Magen für das volumenvergrößernde Material ist länger als für die wirkstoffhaltige Schicht. Durch die Verweilzeit des Materials im Magen wird infolge dessen ein Sättigungsgefühl hervorgerufen und für die Zeit des Verbleibs im Magen erhalten, während die in den Darm gelangten Wirkstoffe dort resorbiert werden.

Erfindungsgemäß ist es daher z.B. möglich in einem Präparat ursächlich das Übergewicht zu therapieren und zugleich symptomatisch das jeweilige Symptom. So kann beispielsweise ein blutdrucksenkender Wirkstoff fest auf das Trägermaterial aufgebracht oder in das Trägermaterial eingebracht werden, das durch die Erzeugung eines Sättigungsgefühls die Nahrungsaufnahme und somit auch das Gewicht reduziert.

Da Gewichtsreduktion zugleich einen erhöhten Blutdruck zu senken vermag, kommt es unter Einsatz des beschriebenen Kombinationspräparats zu einer Wirk- und Therapieverbesserung gegenüber der jeweiligen Einzelgabe.

Erfindungsgemäß ist es aber auch möglich, verschiedene Wirkstoffe zu kombinieren. So kann neben dem beschriebenen im Magen löslichen Haftvermittler ein Haftvermittler zum Einsatz kommen, der sich nicht im Magen löst. Auf diesen Haftvermittler werden sodann Wirkstoffe aufgebracht, die im Magen verbleiben sollen und dort ihre Wirkung entfalten.

Ferner ist es erfindungsgemäß möglich mehrere volumenvergrößernde Materialstücke gleicher oder verschiedener Zusammensetzung miteinander zu verkleben oder in ein Behältnis verbunden oder unverbunden einzubringen. Bei Einsatz des beschriebenen im Magen löslichen Haftvermittlers und/oder der im Magen löslichen Umhüllung lösen sich diese Materialstücke voneinander ab. Hierdurch kann ein erhöhter Sättigungseffekt erreicht werden. Die Wirkstoffe können bei einem solchen System mittels der Haftvermittler zwischen mehreren volumenvergrößernden Materialstücken oder ohne Verbindung mit den Materialstücken in einer Umhüllung angeordnet sein. Bei einem Auseinanderfallen der einzelnen Materialstücke im Magen lösen sich die Wirkstoffschichten ab und gelangen in den Darm, um dort ihre Wirkung zu entfalten. Gegebenenfalls können auch Wirkstoffe zum Einsatz kommen, die sich zumindest teilweise im Magen lösen und gegebenenfalls dort ihre therapeutische Wirkung entfalten. Erfindungsgemäß bevorzugt werden schwammartige volumenvergrößernde Materialien.

Unter solchen schwammartigen volumenvergrößernden Materialien sind erfindungsgemäß feste oder halbfeste elastische Schäume zu verstehen, die aus gasgefüllten beispielsweise polyederförmigen Zellen bestehen, die durch hochviskose und/oder feste Zellstege begrenzt sind. Einsetzbar sind erfindungsgemäß sowohl natürlich vorkommende Schwämme, halbsynthetische oder synthetisch hergestellte schwammartige Gebilde. Beispiele für synthetische schwammartige Materialien sind Polyurethane, Polyacrylate, Poly(met)acrylsäurederivate, Homo- und Copolymere des Vinylacetats. Zu den natürlichen und halbsynthetischen Polymeren zählen u.a. Cellulose, Celluloseether oder Celluloseester wie Celluloseacetat und Celluloseacetatphthalat. Beispiele für natürliche Polymere sind Polysaccharide wie Alginate, Traganth, Xanthan Gumme, Guar Gummi und deren Salze und Derivate. Der Einsatz von Chitin und von Chitinderivaten ist möglich. Im Weiteren werden bevorzugt Stoffe mit Faserstruktur wie Skleroproteine z. B. Collagen, Keratin, Conchagene, Fibroin, Elastin und Chitin eingesetzt. Darüber hinaus sind auch stabil miteinander vernetzte Polysaccharide Gegenstand der vorliegenden Erfindung.

Die Herstellung der schwammartigen bzw.- förmigen Gebilde erfolgt mit an sich bekannten Methoden nach dem Stand der Technik. Hierbei sei auf die WO 98 09 617 verwiesen.

In Abhängigkeit von dem eingesetzten Ausgangsmaterial kann im einfachsten Falle ein Schaum durch Einblasen, durch Schlagen, Schütteln, Verspritzen oder Rühren in der betreffenden Gasatmosphäre erhalten werden. Bei den Polymeren entsteht die Schaumstruktur aufgrund chemischer Reaktionen. So wird z.B. durch Zugabe von Blähmitteln, die sich bei bestimmter Temperatur während der Verarbeitung unter Gasbildung zersetzen, oder durch Zusatz von flüssigen Lösemitteln während der Polymerisation geschäumt. Die Verschäumung erfolgt entweder beim Verlassen des Extrusionswerkzeuges, d.h. im Anschluß an das Extrudieren oder Spritzgießen oder in offenen Formen. Die Härtung erfolgt unter den für die jeweilige chemische Verbindung des Trägermaterials charakteristischen Bedingungen.

Unabdingbare Eigenschaft des erfindungsgemäßen Materials ist, daß es komprimierbar ist. Für dessen Auswahl ist schließlich auch wesentlich, daß es quellfähig bleibt, ohne daß die Zellstege zerstört werden.

Unter physiologischen Bedingungen kann sich das komprimierte Trägermaterial beispielsweise auf das Zwei- bis Zehnfache, bevorzugt auf das Vier- bis Achtfache seines Volumens ausdehnen. Die Wirkstofffreisetzungsflächen des unter physiologischen Bedingungen vergrößerten Materials betragen beispielsweise 15 bis 25 cm². Im Vergleich dazu liegen die Werte der Freisetzungsflächen nach dem Stand der Technik bei 0,5 bis 1,5 cm².

Bei dem erfindungsgemäßen Mittel liegt das volumenvergrößernde Material demgemäß vor und/oder während der Einnahme durch den Patienten bevorzugt in komprimierter Form vor. In einer weiteren Ausführungsform kann das erfindungsgemäße Mittel auch während der Einnahme durch Kau- und/oder Schluckbewegungen komprimiert werden.

Erfindungsgemäß kann auch das volumenvergrößernde Material als solches weitere Hilfsstoffe enthalten.

Hierunter sind beispielsweise folgende Substanzen zu verstehen ; wasserunlösliche Hilfsstoffe oder Gemische davon, wie Lipide, u.a. Fettalkohole, z.B. Cetylalkohol, Stearylalkohol und Cetostearylalkohol; Glyceride, z.B. Glycerinmonostearat oder Gemische von Mono-, Di- und Triglyceriden pflanzlicher Öle; hydrierte Öle, wie hydriertes Rizinusöl oder hydriertes Baumwollsamenöl; Wachse, z.B. Bienenwachs oder Carnaubawachs; feste Kohlenwasserstoffe, z.B. Paraffin oder Erdwachs; Fettsäuren, z.B., Stearinsäure; gewisse Cellulosederivate, z.B. Ethylcellulose oder Acetylcellulose; Polymere oder Copolymere, wie Polyalkylene, z.B. Polyäthylen, Polyvinylverbindungen, z.B. Polyvinylchlorid oder Polyvinylacetat, sowie Vinylchlorid-Vinylacetat-Copolymere und Copolymere mit Crotonsäure, oder Polymere und Copolymere von Acrylaten und Methacrylaten, z.B. Copolymerisate von Acrylsäureester und Methacrylsäuremethylester, verwendet werden.

Außer den genannten Hilfsstoffen können die Mittel gemäß der vorliegenden Erfindung zusätzlich Füll- Spreng-, Binde- und Gleitmittel sowie Trägerstoffe enthalten, die auf die Wirkstoffabgabe keinen entscheidenden Einfluß haben. Beispiele sind u.a. Bentonit (Aluminiumoxid-Siliciumoxid-hydrat), Kieselsäure, Cellulose (üblicherweise mikrokristalline Cellulose) oder Cellulosederivate, z.B. Methylcellulose, Natriumcarboxymethylcellulose, Zucker, wie Lactose, Stärken, z.B. Maisstärke oder Derivate davon, z.B. Natriumcarboxymethylstärke, Stärkekleister, Phosphorsäuresalze, z.B. Di- oder Tricalcioumphosphat, Gelatine, Stearinsäure oder geeignete Salze davon, z.B. Magnesiumstearat oder Calciumstearat, Talk, kollodiales Siliciumoxid und ähnliche Hilfsstoffe.

Auf das beschriebene volumenvergrößernde Material können Wirkstoffe aufgebracht werden, und zwar unter Einsatz der beschriebenen Haftvermittler.

Unter Wirkstoff im Sinne der Erfindung sind alle Stoffe mit einer pharmazeutischen oder biologischen Wirkung zu verstehen. Im folgenden sind Beispiele für erfindungsgemäße wirkstoffhaltige Formulierungen aus unterschiedlichen therapeutischen Klassen wiedergegeben.

Beispiele für ACE-Hemmer sind: Benazepril, Captopril, Cilazapril, Enalapril, Fosinopril, Lisinopril, Perinodopril, Quinapril, Ramipril, Trandolopril.

Beispiele für Analeptika sind: Almitrin, Amiphenazol, Coffein, Doxapram, Etamivan, Fominoben, Metamfetamin, Nicethamid, Pentetrazol.

Beispiele für Analgetika (Opioide) sind: Alfentanil, Buprenorphin, Cetobemidon, Dextromoramid, Dextropropoxyphen, Fentanyl, Flupirtin, Hydromorphon, Levomethadon, Levorphanol, Meptazinol, Morphin, Nalbuphin, Oxycodon, Pentazocin, Pethidin, Piritramid, Tilidin, Tramadol.

Beispiele für Analgetika (Nicht-opioide) sind: Acetylsalicylsäure, Benzylmandelat, Bucetin, Ethenzamid, Ketorolac, Metamizol, Morazon, Paracetamol, Phenacetin, Phenazon, Propyphenazon, Salicylamid.

Beispiele für Anthelminthika sind: Albendazol, Diethylcarbamazin, Mebendazol, Praziquantel, Tiabendazol.

Beispiele für Antiallergika/Antihistaminika sind: Anatazolin , Astemizol, Azelastin, Bamipin, Brompheniramin, Buclizin, Carbinoxamin, Cetririzin, Chlorphenamin, Clemastin, Cyslizin, Cyproheptadin, Dimenhydramin, Doxylamin, Fexofenadin, Ketotifen, Loratadin, Mepyramin, Mizolastin, Nedrocromil, Oxatomid, Oxomemazin, Pheniramin, Phenyltoloxamin, Spagluminsäure, Terfenadin, Triprolidin.

Beispiele für Antiarrhythmmika sind: Ajmalin, Amiodaron, Aprindin, Chinidin, Disopyramid, Mexiletin, Procainamid, Propafenon, Tocainid.

Beispiele für Antibiotika/Chemotherapeutika sind: Amikacin, Gentamicin, Kanamycin,
Paromomycin, Sisomicin, Streptomycin Tobramycin, Chloroquin, Halofantrin, Hydroxychloroquin, Mefloquin, Proguanil, Ethambutol, Isoniazid, Rifabutin, Rifampicin, Cefacetril, Cefaclor, Cefadroxil, Cefalexin, Cefalotin, Cefamandol, Cefazolin, Cefixim, Cefmenoxim, Cefoperazon, Cefotaxim, Cefotetan, Cefotiam, Cefoxitin, Cefpodoxim (proxetil), Cefradin, Cefsulodin, Ceftazidim, Ceftizoxim, Ceftriaxon, Cefuroxim (axetil), Latamoxef, Cinoxacin, Ciprofloxacin, Enoxacin, Nalidixinsäure, Norfloxacin, Ofloxacin, Pipemidsäure, Rosoxacin, Clarithromycin, Erythromycin, Roxithromycin, Amoxicillin, Ampicillin, Apalcillin, Azidocillin, Azlocillin, Bacampicillin, Benzylpenicillin, Carbenicillin, Carindacillin, Dicloxacillin, Flucloxacillin, Mezlocillin, Oxacillin, Phenoxymethypenicillin, Piperacillin, Pivampicillin, Propicillin, Ticarcillin, Colistin, Teicoplanin, Vancomycin, Cotrimoxazol, Sulfametoxydiazin, Doxycyclin, Oxytetracylin, Tetracyclin, Atovaquon, Chloramphenicol, Fosfomycin, Imipenem, Metronidalzol, Nitrofurantoin, Pentamidin, Taurolidin, Trimethoprim.

Beispiele für Antidepressiva sind: Amitripytylin, Amitriptylinoxid, Clomipramin, Desipramin, Dibenzepin, Dosulepin, Doxepin, Fluoxetin, Fluvoyamin, lmipramin, Lithiumsalze, Maprotilin, Nomifensin, Opipramol, Oxitriptan, Tranylcypromin, Trimipramin, Tryptophan.

Beispiele für Antidiabetika / Antihypoglykämika sind: Acarbose, Carbutamid, Chlorpropamid, Glibenclamid, Glibornurid, Gliclazid, Glimepirid, Glipizid, Gliquidon, Glisoxepid, Glymidin, Guar, Insulin, Metformin, Tolazamid, Tolbutamid.

Beispiele für Antidiarrhoika sind: Difenoxin, Diphenoxylat, Loperamid, Petin, Tannin:
Beispiele fürAntidota sind: Flumazenil, Naloxon, Naltrexon.
Beispiele für Antiemetika sind: Alizaprid, Betahistin, Thiethylperazin.
Beispiele für Antiepileptika sind: Barbexaclon, Carbamazepin, Ethosuximid, Lamotrigin, Mepacrin, Mesuximid, Phenobarbital, Phenytoin, Primidon, Sultiam, Trimethadion, Valproinsäure, Vigabatrin.
Beispiele für Antifibrinolytika sind: Aminocapronsäure, 4-(Aminomethyl)benzoesäure, Tranexamsäure.
Beispiele für Antihypertensiva sind: Clonidin, Diazoxid, Doxazosin, Guanethidin, Hydralazin, Methyldopa, Moxonidin, Nitroprussidnatrium, Phentolamin, Prazosin, Reserpin, Tiamenidin, Urapidil.
Beispiele für Antihypotonika sind: Dihydroergotamin, Dobutamin, Dopamin, Etilefrin, Norepinephrin, Norfennefrin.
Beispiele für Antikoagulantia sind: Acenocoumarol, Dalteparin natrium, Enoxaparin, Heparin, Heparinoide Hirudin, Lepirudin, Nadroparin, Parnaparin, Phenprocoumon, Reviparin, Tinzaparin, Warfarin.
Beispiele für Antimykotika sind: Amorolfin, Amphotericin B, Bifonazol, Chlormidazol, Ciclopiroxolamin, Clotrimazol, Croconazol, Econazol, Fenticonalzol, Fluconazol, Griseofulvin, Isoconazol, Itraconazol, Ketoconazol, Miconazol, Naftifin, Naystatin, Omoconazol, Oxiconazol, Terbinafin, Terconazol, Tioconazol, Tolnaftat.
Beispiele für Antirheumatika sind: Acemetacin, Azapropazon, Benorilat, Bumadizon, Carprofen, Cholinsalicylat, Diclofenac, Diflunisal, Etofenamat, Felbinac, Fenbufen, Fenoprofen, Flufenaminsäure, Flurbiprofen, Ibuprofen, Indometacin, Isoxicam, Ketoprofen, Lonazolac, Mefenaminsäure, Meloxicam, Mofebutazon, Nabumeton, Naproxen, Nifenazon, Nifluminsäure, Oxyphenbutazon, Phenylbutazon, Piroxicam, Pirprofen, Proglumetacin, Pyrazinobutazon, Salsalat, Sulindac, Suxibuzon, Tenoxicam, Tiaprofensäure, Tolmetin, Auranofin, Aurothioglucose, Aurothiomalat, Aurothioplypeptid, Chloroquin, Hydroxychloroquin, Penicillamin, Ademetionin, Benzydamin, Bufexamac, Famprofazon, Glucosamin, Oxaceprol.
Beispiele für Antitussiva sind: Benproperin, Butamirat, Butetamat, Clobutinol, Clofedanol, Codein, Dextromethorphan, Dihydrocodein, Hydrocodon, Isoaminil, Natriumdibunat, Noscapin, Oxeladin, Pentoxyverin, Pholcodin, Pipazetat.
Beispiele für Appetitzügler sind: Amfepramon, Fenfluramin, Fenproporex, Levopropylhexedrin, Mazindol, Mefenorex, Metamfepramon, Norephedrin, Norpseudoephedrin.
Beispiele für Beta-Rezeptorenblocker sind: Acebutolol, Alprenolol, Atenolol, Betaxolol, Bisoprolol, Bopindolol, Bupranolol, Carvedilol, Celiprolol, Labetalol, Levobunolol, Mepindolol, Metipranolol, Metoprolol, Nadolol, Oxprenolol, Penbutolol, Pindolol, Propranolol, Sotalol.
Beispiele für Bronchospasmolytika/Antiasthmatika sind: Bambuterol, Carbuterol, Clenbuterol, Epinephrin, Fenoterol, Hexoprenalin, lpratropiumbromid, Isoetarin, Orciprenalin, Oxitropiumbromid, Pirbuterol, Procaterol, Reproterol, Salbutamol, Salmeterol, Terbutalin, Theopohyllin, Tolubuterol.
Beispiele für Calciumantagonisten sind: Amlodipin, Felodipin, Isradipin, Nicardipin, Nifedipin, Nilvadipin, Nitrendipin, Nisoldipin, Verapamil.
Beispiele für Cholagoga sind: Anetholtrithion, Azintamid, Chenodeoxycholsäure, Dehydrocholsäure, Hymecromon, Piprozolin, Ursodeoxycholsäure.
Beispiele für Cholinergika/Cholinolytika sind: Aceclidin, Acetylocholin, Carbachol, Cyclopentolat, Distigmin, Edrophonium, Emepronium, Homatropin, Methanthelin, Neostigmin, Pilocarpin, Propanthelin, Propiverin, Pyridostigmin, Tropicamid.
Beispiele für Diuretika sind: Acetazolamid, Amilorid, Bendroflumethiazid, Bumetanid, Chlorothiazid, Chlortalidon, Clopamid, Etacrynsäure, Furosemid, Hydrochlorothiazid, Triamteren, Xipamid.
Beispiele für Durchblutungsfördernde Mittel / Nootropika sind: Buflomedil, Buphenin, Dextran 40, Dihydroergotoxin, Iloprost, Meclofenoxat, Nicergolin, Nicotinsäure, Pentifyllin, Piracetam, Piribedil, Pyritinol, Tolazolin, Viquidil.
Beispiele für Enzyme / Inhibitoren / Transportproteine sind: Antithrombin III, Aprotinin, Carnitin, Clavulansäure, Dornase alfa, Sulbactan.
Beispiele für Expektorantia sind: Acetylcystein, Ambroxol, Bromhexin,Carbocistein, Colfosceril, Surfactant (aus Rinderleber), Surfactant (aus Schweinelunge).
Beispiele für Gichtmittel sind: Allopurinol, Benzbromaron, Colchicin, Probenecid, Sulfinpyrazon.
Beispiele für Glukokortikoide sind: Betamethason, Budesonid, Cloprednol, Cortison, Dexamethason, Flunisolid, Fluticason, Hydrocortison, Methylprednisolon, Paramethason, Prednisolon, Prednison, Prednyliden, Triamcinolon.
Beispiele für Hämostyptika sind: Adrealon, Blutgerinnungsfaktor VII, Blutgerinnungsfaktor VIII, Blutgerinnungsfaktor IX, Blutgerinnungsfaktor XIII, Carbazochrom, Etamsylat, Fibrinogen, Kollagen, Menadiol, Menadion, Protamin, Somatostain, Thrombin, Thromboplastin.
Beispiele für Hypophysen-/Hypothalamus-Hormone und -Hemmstoffe sind: Argipressin, Choriongonadotrophin, Desmopressin, Felypressin, Gonadorelin, Lypressin, Menotropin, Ornipressin, Quinagolid, Terlipressin, Thyrotrophin.
Beispiele für Immuntherapeutika und Zytokine sind: Aldesleukin, Azathioprin, BCG, Ciclosporin, Filgrastim, Interferon alfa, Interferon beta, Interleukin-2, Muromonab-CD3, Tacrolismus, Thymopentin, Thymostimulin.
Beispiele für Kardiaka sind:Acetyldigitoxin, Acetylödiagoxin, Convallatoxin, Digitoxin, Digoxin, Gitoforrnat, Lanatosid, Meproscillarin, Metildigoxin, Pengitoxin, Peruvosid, Proscillaridin, Strophanthin, Thevetin, Amrinon, Enoximon, Milrinon,
Beispiele für Koronarmittel sind: Carbocromen, Isosorbiddinitrat, Nitroglycerin, Pentaerythrityltetranitrat.
Beispiele für Laxantia sind: Bisacodyl, Dantron, Docusat, Glycerol, Lactulose, Magnesiumsulfat, Natriumpicosulfat, Natriumsulfat, Paraffinum subliqiudum, Phenolphthalein, Rizinusöl, Sorbitol.
Beispiele für Lebertherapeutika sind: Cholin, Citiolon, Myo-Inositol, Silymarin.
Beispiele für Lipidsenker sind: Acipimox, Bezafibrat, Clofibrat, Etofibrat, Fluvastin, Lovastatin, Pravastatin, Simvastin.
Beispiele für Lokalanästhetika sind: Articain, Benzocain, Bupivacain, Butanilicain, Chlorethan, Cinchocain, Cocain, Etidocain, Fomocain, Lidocain, Mepivacain, Myrtecain, Oxetacain, Oxybuprocain, Polidocanol, Prilocain, Procain, Proxymetacain, Quinisocain, Tetracain.
Beispiele für Magen- /Darm-Mittel sind: Bismutsubcitrat, Bromoprid, Garbenoxolon, Cimetidin, Domperidon, Famotidin, Metoclopramid, Nizatidin, Omeprazol, Proglumid, Ranitidin, Roxatidin, Sucralfat, Sulfasalazin.
Beispiele für Migränemittel sind: Ergotamin, Lisurid, Naratriptan, Pizotifen, Sumatriptan, Zolmitriptan.
Beispiele für Muskelrelaxantia sind: Alcuronium, Atracurium, Baclofen, Carisoprodol, Chlormezanon, Clostridiumtoxin botulinum Toxin A,
Beispiele für Nebenschilddrüsen-Therapeutika/Calciumstoffwechsel-Regulatoren sind: Clodronsäure, Dihydrotachysterol, Glandulae parathyreoideae, Pamidronsäure.
Beispiele für Neuroleptika sind: Benperidol, Chlorpromazin, Droperidol, Flugheanzin, Haloperidol, Melperon, Promethazin, Zuclopenthixol.
Beispiele für Parkinson-Mittel sind: Amantadin, Benserazid, Benzatropin, Biperiden, Bornaprin, Bromocriptin, Cabergolin, Carbidopa, Diphydroergocriptin, Levodopa, Metixen, Pergolid, Pramipexol, Ropinirol, Tolcapon.
Beispiele für Psychostimulantia sind: Amfetaminil, Deanol, Fencamfamin, Fenetyllin, Kavain, Methylphenidat, Pemolin, Prolintan.
Beispiele für Schilddrüsen-Therapeutika sind: Carbimazol, Glandulae thyreoideae, Jod, Jodid, Levothyroxin, Liothyronin, Methylthiouracil, Perchlorat, Proloniumjodid, Propylthiouracil, Radio-Jod, Thiamazol.
Beispiele für Sedativa/Hypnotika sind: Amobarbital, Chloralhydrat, Clomethiazol, Glutethimid, Hexobarbital, Methaqualon, Methyprylon, Pentobarbital, Scopolamin, Secbutabarbital, Secobarbital, Vinylbital, Zolpidem, Zopiclon.
Beispiele für Sexualhormone sind: Chlorotrianisen, Clomifen, Clostebol, Cyproteron, Drostanolon, Epimestrol, Estradiol, Estriol, Estron, Ethinylestradiol, Flutamid, Fosfestrol, Konjugierte Estrogene, Medroxyprogesteron, Mesterolon, Mestranol, Metenolon, Methyltestosteron, Nandrolon, Oxymetholon, Polyestradiophosphat, Quinestrol, Stanozolol, Testosteron.
Beispiele für Spasmolytika sind: Atropin, Butylscopolamin, Flavoxat, Glycopyrronium, Mebeverin, Methylscopolamin, Oxybutynin, Tiropramid, Trospiun.
Beispiele für Thrombozytenaggregationshemmer sind: Abciximab, Acetylsalicylsäure, Dipyridamol, Ticlopidin.
Beispiele für Transquilizer sind: Alprazolam, Bromazepam, Brotizolam, Buspiron, Camazepam, Chlordiazepoxid, Clobazam, Clonazepam, Clorazepat, Clotiazepam, Diazepam, Flunitrazepam, Flurazepam, Hydroxyzin, Ketazolam, Loprazolam, Lorazepam, Lormetazepam, Medazepam, Meprobamat, Metaclazepam, Midazolam, Nitrazepam, Oxazepam, Oxazolam, Prazepam, Temazepam, Tetrazepam, Triazolam.
Beispiele für Urologika sind: Finasterid.
Beispiele für Varia sind: Dapiprazol, Diethyltoluamid, Liponsäure.
Beispiele für Venenmittel sind: Aescin, Calcium dobesilat, Cumarin, Diosmin, Rutosid, Troxerutin.
Beispiele für Virustatika sind: Aciclovir, Cidofovir, Didanosin, Famciclovir, Foscamet, Ganciclovir, Lamivudin, Ritonavir, Zalcitabin, Zidovudin.
Beispiele für Vitamine sind: Alfacalcidol, Allithiamine, Ascorbinsäure, Biotin, Calcifediol, Calcitriol, Colecalciferol, Cyanocobalamin, Ergocalciferol, Folsäure, Hydroxocobalamin, Nicotinamid, Pantothensäure, Phytomenadion, Pyridoxin, Retinol, Riboflavin, Thiamin, Tocopherol, Transcalcifediol.
Beispiele für Zytostatika sind: Aclarubicin, Altretamin, Aminoglutethimid, Amsacrin, Asparaginase, Bleomycin, Buserelin, Busulfan, Carboplatin,Carmustin, Chlorambucil, Cladribin, Cisplatin, Cyclophosphamid, Cytarabin, Dacarbazin, Daunorubicin, Diethylstilbestrol, Docetaxel, Doxorubicin, Epirubicin, Etoposid, Fludarabin, Fluorouracil, Gemcitabin, Goserelin, Hydroxycarbamid, Idarubicin, Ifosfamid, Lomustin, Melphalan, Mercaptopurin, Mesna, Methotrexat, Miltefosin, Mitomycin,
   Mitoxantron, Panorex, Paclitaxel, Plicamycin, Tamoxifen, Tegafur, Thiotepa, Tioguanin, Topotecan, Triptorelin, Vinblastin, Vincristin, Vindesin, Zorubicin.

Die genannten Wirkstoffe liegen erfindungsgemäß in Form eines Pulvers, einer Paste, einer Folie, als Lösung, als Schmelze vor und werden auf das Trägermaterial unter Einsatz der bezeichneten Haftvermittler aufgebracht. Die beschriebenen Wirkstoffe können zusätzlich auch in das Trägermaterial eingebracht sein. U.U. kann hier zusätzlich eine retardierende Wirkstofffreisetzung erzielt werden.

Wie bereits oben dargelegt, wird durch die Auswahl der vorzugsweise eingesetzten Haftvermittler und/oder der Umhüllung sichergestellt, daß sich die volumenvergrößernden Materialien und Wirkstoffe im Magen voneinander lösen und gegebenenfalls direkt in den Darm gelangen. Daneben können weitere Wirkstoffschichten vorhanden sein, die ggf. über Haftvermittler mit der jeweils darunter liegenden Wirkstoffschicht verbunden sind. Auf diese Art kann eine Kombination von Wirkstoffen erreicht werden, sich unter den Bedingungen des Magenmilieus lösen und gegebenenfalls direkt im Darm ihre Wirkung zu entfalten vermögen, während das volumenvergrößernde Material im Magen verbleibt und dort einen Sättigungseffekt bewirkt. Je nach Art des Wirkstoffs ist es aber auch möglich, daß dieser sich zumindest teilweise im Magen löst und so nur teilweise oder gar nicht in den Darm gelangt. Bei Beaufschlagung des volumenvergrößernden Materials durch zusätzliche Wirkstoffe sind weitere beliebige Behandlungen durchführbar. U.a. kann bei retardierender Freisetzung eine Depotwirkung zusätzlich erzielt werden. Ebenso sind mehrschichtige Wirkstoffaufbauten denkbar, bei denen teilweise die Haftvermittler sich im Magen lösen und teilweise nicht lösen.

Auf diese lassen sich beliebige Wirkstoffkombinationen herstellen, die z.T. im Magen und z.T. im Darm ihre Wirkung entfalten.

Gegenstand der vorliegenden Erfindung ist ferner ein Verfahren zur Herstellung des erfindungsgemäßen Mittels, wobei das volumenvergrößernde Material nach bekannten Methoden hergestellt und auf wenigstens einen Teil der Oberfläche des volumenvergrößernden Materials der Haftvermittler und die wirkstoffhaltige Formulierung auf den Haftvermittler aufgebracht wird.

Alternativ kann der Haftvermittler auch auf die wirkstoffhaltige Schicht insbesondere wenn diese ein freitragender Film ist, aufgetragen und erst dann die Verbindung zum Trägermaterial hergestellt werden.

Das erfindungsgemäße Verfahren zeichnet sich ferner dadurch aus, daß die verbindende Schicht beispielsweise in Form eines Pulvers, einer Paste, einer Folie, als Lösung und/oder als Schmelze auf das schwammartige Material und/ oder die wirkstoffhaltige Schicht aufgebracht wird. Hierbei kann die verbindende Schicht beispielsweise durch Sprüh-, Streich-, Tauch- oder Schmelzvorgänge oder andere geeignete Arbeitsschritte auf die entsprechenden Schichten aufgebracht werden. Das volumenvergrößernde Material und der Wirkstoff können durch eine äußere Umhüllung, z. B. eine Beschichtung oder ein Behältnis verbunden werden. Das Aufbringen der Beschichtung kann durch Sprühen, Streichen, Tauchen oder Aufbringen von Schmelzen erreicht werden.

Erfindungsgemäß können auch erfindungsgemäß eine oder mehrere Wirkstoffe aufgebracht werden, die Haftvermittler enthalten. Zum Aufbringen der Haftvermittler können geeignete physikalische Bezeichnungen gewählt werden. Zum Beispiel können der Druck und/oder die Temperatur erhöht werden, um die Kleber z.B. zu erweitern. Ebenso ist es möglich, den Wirkstoff oder die Wirkstoffe unter erhöhtem Druck oder erhöhter Temperatur aufzubringen. Schließlich können sowohl Haftvermittler als auch Wirkstoff zugleich oder nacheinander unter erhöhtem Druck oder Temperatur aufgebracht werden. Erfindungsgemäß ist es ferner möglich Wirkstoffe einzusetzen, denen zuvor keine spezielle Haftvermittler zugesetzt wurden, die aber nach Druck- und/oder Temperatureinwirkung auf dem volumenvergrößernden Material haften.

Erfindungsgemäß kann in einem weiteren Schritt des Verfahrens die als Umhüllung bezeichnete weitere, äußere Schicht aufgebracht werden, die weitere Hilfsstoffe enthalten kann, wie beispielsweise Verbindungen, die das Schlucken oder Einnehmen des erfindungsgemäßen Mittels erleichtern und dem Fachmann unter "Coating"-Verbindungen oder als Dragiermittel bekannt sind. Diese Umhüllungen können zusätzlich oder alternativ zu den beschriebenen Haftvermittlern zur Herstellung der löslichen Verbindung der volumenvergrößernden Materialien und der wirkstoffhaltigen Formulierungen dienen.

Gegenstand der vorliegenden Erfindung ist ferner die Verwendung des erfindungsgemäßen Mittels zur Herstellung eines Mittels zur Behandlung von Übergewicht in Kombination mit mindestens einer weiteren Indikation.

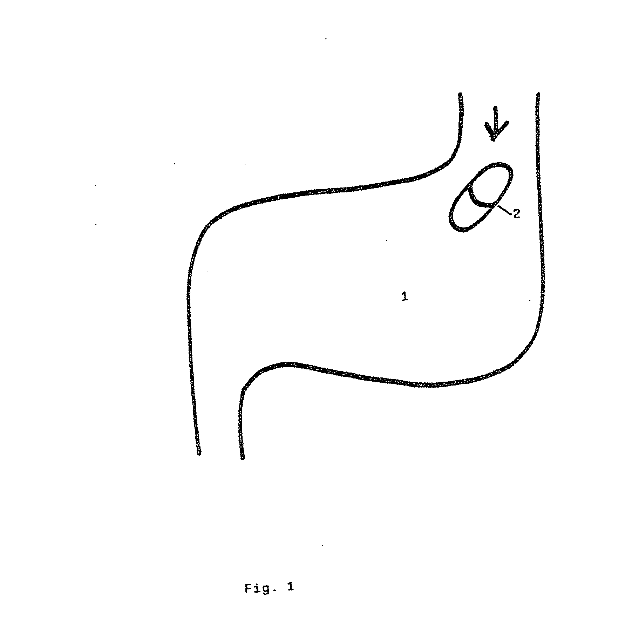

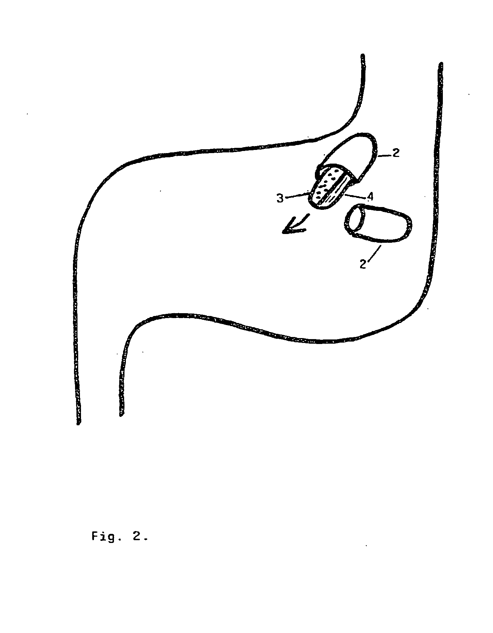

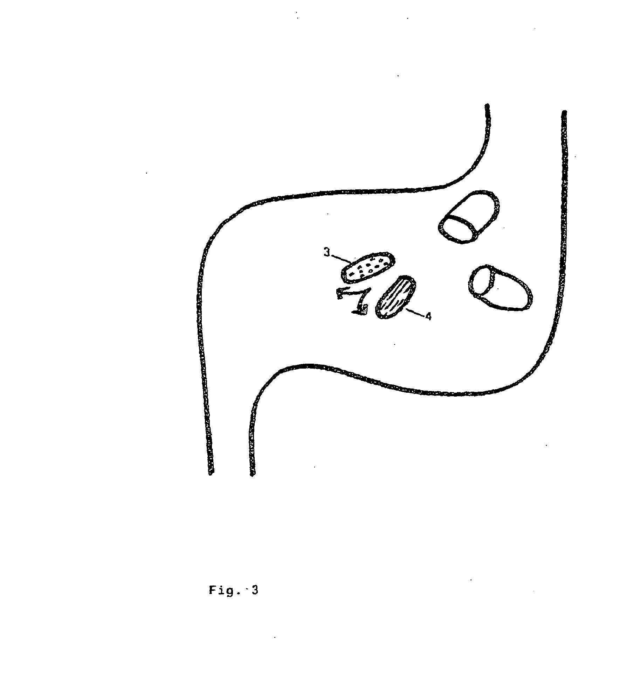

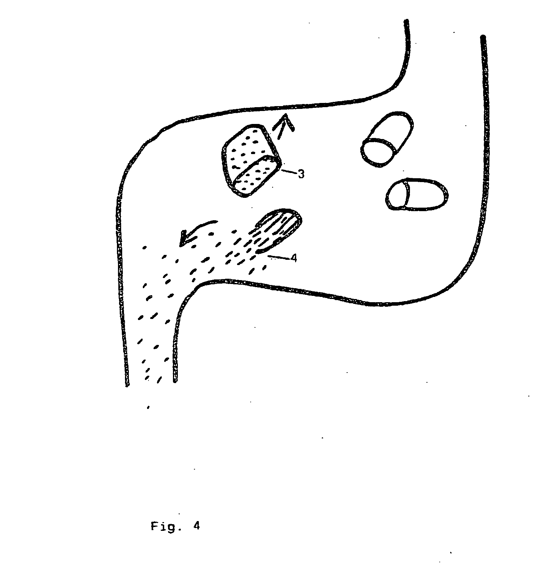

Die vorliegende Erfindung wird durch die Figuren näher erläutert Gemäß Figur 1 wird im Magen 1 eine Kapsel 2, welche volumenvergrößerndes Material 3 und Wirkstoffe 4 enthält, eingebracht. Gemäß 2 beginnt die Kapsel 2 die Inhaltsstoffe 3 und 4 freizugeben. In Figur 3 liegen diese Inhaltsstoffe 3 und 4 frei im Magen vor. In Figur 4 ist zu erkennen, daß das volumenvergrößernde Material aufgequollen ist. Die freigesetzten Wirkstoffe 4 werden zum Darm transportiert, während das in seinem Volumen vergrößerte Material 3 im Magen verbleibt.

## Patentansprüche

1. Mittel zur Behandlung von Übergewicht in Kombination mit mindestens einer weiteren Indikation enthaltend ein in gastrointestinalen Flüssigkeiten und/oder Körperflüssigkeiten unlösliches oder schwer lösliches, volumenvergrößerndes Material und wenigstens eine wirkstoffhaltige im Darm resorbierbare Formulierung, die mittels eines im Magen zumindest teilweise löslichen Haftvermittlers derart miteinander verbunden sind, daß
a) unter den Milieubedingungen im Magen das Material und die Formulierung voneinander getrennt werden,
b) aufgrund dessen die Formulierung in den Darm gelangt, während das volumenvergrößernde Material im Magen verbleibt, wobei
als Haftvermittler Stärkederivate, Cellulosederivate, Gelatine oder Polysaccharide eingesetzt werden,
als volumenvergößerndes Material feste oder halbfeste elastische Schäume, die aus gasgefüllten Zellen bestehen, die durch hochviskose und/oder feste Zellstege begrenzt sind, ausgewählt aus der Gruppe bestehend aus natürlich vorkommenden Schwämme, halbsynthetischen oder synthetisch hergestellten schwammartigen Gebilden, eingesetzt werden, und
als im Darm resorbierbare Formulierung wirkstoffhaltige Formulierungen, enthaltend Wirkstoffe in Form eines Pulvers, einer Paste, einer Folie, als Lösung, als Schmelze, eingesetzt werden, und wobei
durch Beaufschlagen der wirkstoffhaltigen Formulierung und/oder des Trägermaterials mit dem Haftvermittler eine Verbindung zwischen volumenvergrößerndem Material und der wirkstoffhaltigen Schicht erzielt wird, die Haftvermittlerschicht folienförmig ausgestaltet ist und/oder der Haftvermittler fadenförmige und/oder gewebeähnliche Stoffe enthält.

2. Mittel gemäß Anspruch 1, **dadurch gekennzeichnet, daß** das volumenvergrößernde Material in komprimierter Form vorliegt.

3. Mittel gemäß einem der Ansprüche 1 oder 2, **dadurch gekennzeichnet, daß** die wirkstoffhaltige Formulierung übliche Hilfsstoffe und Additive aufweist.

4. Mittel nach einem der Ansprüche 1 bis 3, **dadurch gekennzeichnet, daß** es mit einer Umhüllung versehen ist.

5. Mittel nach Anspruch 4, **dadurch gekennzeichnet, daß** die Umhüllung eine auf das Volumen vergrößernde Material und die wirkstoffhaltige Formulierung aufgebrachte Beschichtung ist.

6. Mittel nach Anspruch 5, **dadurch gekennzeichnet, daß** die Beschichtung ein folienhaltiges Material ist.

7. Mittel nach Anspruch 6, **dadurch gekennzeichnet, daß** das folienartige Material Polymere, Faserstoffe oder Gemische dieser Stoffe enthält.

8. Mittel nach Anspruch 6, **dadurch gekennzeichnet, daß** die Umhüllung als Behältnis ausgestaltet ist.

9. Mittel nach Anspruch 8, **dadurch gekennzeichnet, daß** die Behältnisse kapselförmig ausgestaltet sind.

10. Verfahren zur Herstellung eines Mittels nach einem der Ansprüche 1 bis 3, **dadurch gekennzeichnet, daß** das volumenvergrößernde Material nach bekannten Methoden hergestellt und auf wenigstens einen Teil der Oberfläche des volumenvergrößernden Materials der Haftvermittler und die wirkstoffhaltige Formulierung auf den Haftvermittler aufgebracht wird.

11. Verfahren gemäß Anspruch 10, **dadurch gekennzeichnet, daß** ein oder mehrere mindestens einen Haftvermittler enthaltender Wirkstoff(e) eingesetzt werden.

12. Verfahren zur Herstellung eines Mittels nach einem der Ansprüche 4 bis 9, **dadurch gekennzeichnet, daß** das volumenvergrößernde Material und die wirkstoffhaltige Formulierung getrennt hergestellt über einen Haftvermittler verbunden und anschließend mit einer gemeinsamen äußeren Umhüllung versehen werden.

13. Verwendung eines Mittels gemäß einem der Ansprüche 1 bis 9 zur Herstellung eines Mittels zur Behandlung von Übergewicht in Kombination mit mindestens einer weiteren Indikation.

## Claims

1. Composition for treatment of obesity in combination with at least one further indication, comprising a volume-increasing material which is insoluble or sparingly soluble in gastrointestinal fluids and/or body fluids and at least one active ingredient-containing formulation absorbable in the intestine, which are bonded to one another by means of an adhesion promoter which is at least partly soluble in the stomach, in such a way that
a) under the conditions in the stomach, the material and the formulation are separated from one another,
b) as a result of which the formulation passes into the intestine, while the volume-increasing material remains in the stomach, wherein
the adhesion promoters used are starch derivatives, cellulose derivatives, gelatins or polysaccharides,
the volume-increasing materials used are solid or semisolid elastic foams which consist of gas-filled cells which are bounded by high-viscosity and/or solid cell walls selected from the group consisting of naturally occurring sponges, semisynthetic or synthetic sponge-like structures, and
the formulations absorbable in the intestine are active ingredient-containing formulations comprising active ingredients in the form of a powder, of a paste, of a film, as a solution, as a melt, and wherein
contacting the active ingredient-containing formulation and/or the carrier material with the adhesion promoter achieves a bond between volume-increasing material and the active ingredient-containing layer, the adhesion promoter layer is configured in the form of a film and/or the adhesion promoter contains fibrous and/or fabric-like substances.

2. Composition according to Claim 1, **characterized in that** the volume-increasing material is in compressed form.

3. Composition according to either of Claims 1 and 2, **characterized in that** the active ingredient-containing formulation comprises customary assistants and additives.

4. Composition according to any of Claims 1 to 3, **characterized in that** it has been provided with a casing.

5. Composition according to Claim 4, **characterized in that** the casing is a coating applied to the volume-increasing material and the active ingredient-containing formulation.

6. Composition according to Claim 5, **characterized in that** the coating is a film-containing material.

7. Composition according to Claim 6, **characterized in that** the film-type material comprises polymers, fibrous substances or mixtures of these substances.

8. Composition according to Claim 6, **characterized in that** the casing is configured as a receptacle.

9. Composition according to Claim 8, **characterized in that** the receptacles are configured in the form of capsules.

10. Process for producing a composition according to any of Claims 1 to 3, **characterized in that** the volume-increasing material is produced by known methods and the adhesion promoter is applied to at least part of the surface of the volume-increasing material, and the active ingredient-containing formulation to the adhesion promoter.

11. Process according to Claim 10, **characterized in that** one or more active ingredient(s) comprising at least one adhesion promoter are used.

12. Process for producing a composition according to any of Claims 4 to 9, **characterized in that** the volume-increasing material and the active ingredient-containing formulation are produced separately, bonded by means of an adhesion promoter and then provided with a common outer casing.

13. Use of a composition according to any of Claims 1 to 9 for producing a composition for treatment of obesity in combination with at least one further indication.

## Revendications

1. Agent pour le traitement de la surcharge pondérale en combinaison avec au moins une autre indication contenant un matériau insoluble ou peu soluble dans des liquides gastro-intestinaux et/ou des liquides corporels, augmentant de volume et au moins une formulation contenant une/des substances actives, résorbable dans l'intestin, qui sont liés l'un à l'autre au moyen d'un promoteur d'adhérence au moins partiellement soluble dans l'estomac, de manière telle que
a) dans les conditions environnementales dans l'estomac, le matériau et la formulation sont séparés l'un de l'autre,
b) suite à quoi la formulation arrive dans l'intestin, alors que le matériau augmentant de volume reste dans l'estomac, où
on utilise comme promoteur d'adhérence des dérivés d'amidon, des dérivés de cellulose, de la gélatine ou des polysaccharides,
on utilise comme matériau augmentant de volume des mousses solides ou semi-solides élastiques, qui sont constituées de cellules remplies de gaz, qui sont limitées par des âmes de cellule hautement visqueuses et/ou solides, choisies dans le groupe constitué par les éponges naturelles, les structures de type éponge préparées de manière semi-synthétique ou synthétique, et
on utilise comme formulation résorbable dans l'intestin des formulations contenant une/des substances actives sous forme d'une poudre, d'une pâte, d'une feuille, sous forme de solution, sous forme de masse fondue et où
on obtient, en chargeant la formulation contenant une/des substances actives et/ou le matériau support avec le promoteur d'adhérence, un assemblage entre le matériau augmentant de volume et la couche contenant la/les substances actives, la couche de promoteur d'adhérence est réalisée sous forme de feuille et/ou le promoteur d'adhérence contient des substances en forme de fil et/ou analogues à des tissus.

2. Agent selon la revendication 1, **caractérisé en ce que** le matériau augmentant de volume se trouve sous forme comprimée.

3. Agent selon l'une quelconque des revendications 1 ou 2, **caractérisé en ce que** la formulation contenant une/des substances actives contient des adjuvants et des additifs usuels.

4. Agent selon l'une quelconque des revendications 1 à 3, **caractérisé en ce qu'**il est pourvu d'un enrobage.

5. Agent selon la revendication 4, **caractérisé en ce que** l'enrobage est un revêtement appliqué sur le matériau augmentant de volume et la formulation contenant une/des substances actives.

6. Agent selon la revendication 5, **caractérisé en ce que** le revêtement est un matériau contenant une feuille.

7. Agent selon la revendication 6, **caractérisé en ce que** le matériau de type feuille contient des polymères, des substances fibreuses ou des mélanges de ces substances.

8. Agent selon la revendication 6, **caractérisé en ce que** l'enrobage est réalisé sous forme d'un conteneur.

9. Agent selon la revendication 8, **caractérisé en ce que** les conteneurs sont réalisés sous forme de capsules.

10. Procédé pour la préparation d'un agent selon l'une quelconque des revendications 1 à 3, **caractérisé en ce que** le matériau augmentant de volume est fabriqué selon des procédés connus et le promoteur d'adhérence est appliqué sur au moins une partie de la surface du matériau augmentant de volume et la formulation contenant une/des substances actives est appliquée sur le promoteur d'adhérence.

11. Procédé selon la revendication 10, **caractérisé en ce qu'**on utilise une ou plusieurs substances actives contenant au moins un promoteur d'adhérence.

12. Procédé pour la préparation d'un agent selon l'une quelconque des revendications 4 à 9, **caractérisé en ce que** le matériau augmentant de volume et la formulation contenant la/les substances actives sont préparés séparément, assemblés via un promoteur d'adhérence et ensuite pourvus ensemble d'un enrobage externe.

13. Utilisation d'un agent selon l'une quelconque des revendications 1 à 9 pour la préparation d'un agent destiné au traitement de la surcharge pondérale en combinaison avec au moins une autre indication.
